# EUROPÄISCHE PATENTSCHRIFT

(11) **EP 1 946 719 B1**
(45) Veröffentlichungstag und Bekanntmachung des Hinweises auf die Patenterteilung: **22.06.2011**
(21) Anmeldenummer: 08006951.1
(22) Anmeldetag: 04.04.2007
(51) Int. Cl.: A61B 19/02, A61C 19/02, A61L 2/26

(54) **Lagerungssystem für Implantate und Schraubenverpackungen**
Storage system for implants and screw packaging
Système de stockage pour implants et emballages de vis

(30) Priorität: 07.04.2006 DE 102006016866
(43) Veröffentlichungstag der Anmeldung: 23.07.2008
(62) Teilanmeldung aus: 07007013.1
(73) Patentinhaber: MEDICON EG CHIRURGIEMECHANIKER-GENOSSENSCHAFT, D-78532 Tuttlingen (DE)
(72) Erfinder: Burger, Andreas, 78532 Tuttlingen (DE); Wenzler, Klaus, 78665 Frittlingen (DE); Schröder, Kristine, 78567 Fridingen (DE); Hettich, Petra, 78665 Frittlingen (DE); Liebermann, Karl-Ernst, 78570 Mühlheim (DE)
(74) Vertreter: Mammel, Ulrike

(56) Entgegenhaltungen:
- DE-A1- 10 230 519
- US-A- 5 411 136
- US-A- 5 451 380
- US-A- 5 893 618
- US-A- 6 116 452
- US-A- 6 138 850
- US-A1- 2003 198 581
- US-B1- 6 783 004

## Beschreibung

Die vorliegende Erfindung betrifft ein Lagerungssystem für Implantate und Schraubenverspackungen.

Lagerungssysteme können beispielsweise in der Traumatologie, der Mund-, Kiefer-, Gesichts-Chirurgie, der Hals-Nasen-Ohren-Chirurgie, der Neurochirurgie, der Schädelbasis-Chirurgie, der Handchirurgie und der okuloplastischen Chirurgie eingesetzt werden.

Aus dem Stand der Technik sind Lagerungssysteme der Gebrüder Martin GmbH & Co. KG bekannt, die neben dem Boden mit Öffnungen zum Durchtritt der Reinigungsflüssigkeit und Öffnungen im Boden zur Aufnahme der Schraubenverpackungen starre vollflächige Seitenwände aufweisen. Von oben können die Lagerungssysteme mit einem aufschieb- oder aufclipsbaren Deckel, der ebenfalls eine Vielzahl kleiner Öffnungen zum Durchtritt der Reinigungsflüssigkeit und des Dampfes aufweist, verschlossen werden.

Wahlweise können in diese Lagerungssysteme weitere Einsätze, beispielsweise für Biegeschablonen, Knochenplatten oder zur Instrumentenlagerung eingebracht werden. Auch diese Einsätze sind kastenförmig mit starren Wänden und einer Lochbodenplatte ausgestattet. Die Einsätze werden von oben in die Öffnung bzw. bei Aufteilung des Lagerungssystems zur Lagerung zweier Einsätze in eine der Öffnungen eingesetzt.

Solche kastenförmigen Lagerungssysteme mit einem Lochbodenblech, starren Wänden und einem Deckel, die zur Aufnahme von Implantaten und/oder Instrumenten dienen, in welchen die Implantate oder Instrumente für die jeweilige OP zusammengestellt und anschließend gereinigt und sterilisiert werden, sind auch von einer Vielzahl von anderen Herstellern bekannt.

Nachteilig ist an diesen Lagerungssystemen, dass der Zutritt der Reinigungsflüssigkeit zu den Implantaten und Instrumenten behindert ist. Zudem ist es bei den bekannten Lagerungssystemen möglich, dass die Sterilisation bei 5 Minuten und 134° C (Standardbedingungen) nicht ausreichend ist. Für diese Systeme muss die Sterilisationszeit individuell angepasst werden, was zusätzlichen Aufwand und eine Fehlerquelle darstellt.

Aus der DE 697 24 045 T2 ist ein Behälter bekannt, der zum Sterilisieren von chirurgischen Instrumenten dient, wobei die chirurgischen Instrumente in dem Sterilisierbehälter durch Haltevorrichtungen gehalten werden. Zur Lagerung von chirurgischen Instrumenten, Implantaten und Schraubverpackungen ist dieser Behälter nicht vorgesehen. Der Sterilisierbehälter umfasst einen Träger, eine Matte mit Halteeinrichtungen, Ablauföffnungen und einen Deckel, wobei der Träger massive Abschluss- und Seitenwände umfasst. In den Seitenwänden sind am oberen Ende flache Ausschnitte vorgesehen, die mit entsprechenden flachen Ausschnitten in der Seitenwand des Deckels korrespondieren, so dass flache schlitzförmige Öffnungen in dem geschlossenen Behälter gebildet werden.

Aus der US 6,116,452, von der der Gegenstand des Anspruchs 1 abgeleitet wird, ist ein Lagerungssystem mit einer Bodenplatte, Seitenflächen und einem Deckel bekannt. An den beiden Kopfseiten sind Freischnitte in den Seitenflächen vorgesehen, die im geschlossenen Zustand des Lagerungssystems weitgehend von entsprechend geformten Laschen an dem Deckel des Lagerungssystems bedeckt werden.

Auch aus der US 6,138,850 ist ein Lagerungssystem mit Boden, Seitenflächen und einem Deckel bekannt. Dieser Lagerungssysteme weisen ebenfalls Freischnitte in den beiden Kopfteilen auf, die zur Aufnahme entsprechend geformter Handgriffe an dem Deckel dienen.

Aus der US 5,893 618 A ist ein stapelbarer Sterilisierbehäiter bekannt, der ein Rack umfasst und verschiedene schubladenartige Ablagekästen, die zur Lagerung der chirurgischen Instrumente dienen. Die Seitenwände des Racks sind teilweise offen.

Die Aufgabe der vorliegenden Erfindung besteht darin, Lagerungssysteme für Implantate bereitzustellen, die sich durch eine einfachere Handhabbarkeit und bessere Reinig- und Sterilisierbarkeit auszeichnen.

Diese Aufgabe wird dadurch gelöst, dass die Hauptabschnitte und ggf. seitlich vorhandenen Nebenabschnitte zusammen mit dem Boden oder einer an dem Boden befestigten Stütze einen Rahmen oder eine Tragbalkenkonstruktion bildern und das Lagerungssystems einen tischartigen Einsatz zur Aufnahme von Verpackungen von Schrauben, Nägeln, Aneurysmenclips oder Gefäßclips umfasst und der tischartige Einsatz eine horizontale Platte mit mehreren länglichen Öffnungen und wenigstens vier Füße aufweist, wobei durch die Öffnung oder den Freischnitt zwischen den beiden benachbarten Hauptabschnitten eine seitliche Zugänglichkeit und ein guter Durchtritt der Reinigungsflüssigkeit in der Spülmaschine und des Dampfes selbst dann gewährleistet ist, wenn der tischartige Einsatz in dem Lagerungssystem angeordnet ist.

Das neuartige Lagerungssystem weist nicht - wie aus dem Stand der Technik hinreichend bekannt - massive Seitenwände auf, sondern vielmehr wenigstens vier senkrecht zur Bodenplatte verlaufende stegförmige Hauptabschnitte, die als Führungsflächen für die innerhalb der Halteabschnitte angeordnete Einlegeböden, Einlegetische, Tabletts, Unterteilungen etc. dienen können, wobei durch die große Öffnung oder den großen Freischnitt zwischen zwei Hauptabschnitten ein sehr guter Durchtritt der Reinigungsflüssigkeit in der Spülmaschine und des Dampfes gewährleistet ist.

Je mehr offene oder freigeschnittene Flächen das erfindungsgemäße Lagerungssystem aufweist, desto besser gelangen die Reinigungsflilssigkeit und der Dampf an die innerhalb des Systems lagernden Implantate und Instrumente. Bevorzugt sind wenigstens zwei, besonders bevorzugt jeweils einander gegenüberliegende Seiten offen oder freigeschnitten. Der Prozentsatz der offenen oder freigeschnittenen Seitenflächen zur gesamten Seitenfläche des Lagerungssystems sollte wenigstens 30 %, vorzugsweise wenigstens 40 % und besonders bevorzugt mehr als 50 % betragen, insbesondere wenigstens 65 %.

Soweit das vorzugsweise im Wesentlichen rechteckige Lagerungssystem nicht nur an jeder Ecke, sondern auch noch an den Längsseiten zwischen zwei Ecken einen zusätzlichen Nebenabschnitt aufweist, sind in einer besonders bevorzugten Ausführungsform jeweils die beiden gegenüberliegenden Längsseiten bis auf die Haupt- und Nebenabschnitte und gegebenenfalls eine Schiene, an der die Bodenplatte befestigt ist und an der die Halteabschnitte angeordnet sind, offen bzw. fretgeschrtitten.

Die Haupt- und Nebenabschnitte weisen auf ihrer nach innen weisenden Seite Führungsflächen zum Einlegen von Einlegeböden, Tabletts, Eintegetischen etc. auf. Diese Einlegeböden, Tabletts, Einlegetische etc. können verschiedenartig gestaltet sein, beispielsweise Ausnehmungen für Knochenplatten aufweisen oder kastenförmig ausgebildet sein, um darin Instrumente aufzunehmen.

Sind an der Längsseite In der freigeschnittenen Fläche zusätzliche Nebenabschnitte vorgesehen, so weisen diese lediglich eine Stützfunktion für den Deckel und eine Lagesicherung für Einlegeböden, Tabletts, Einlegetische etc, auf. Die Hauptabschnitte an der Ecke und gegebenenfalls die Nebenabschnitte an der Längsseite bilden einen Rahmen bzw. eine Tragstruktur oder Tragbalkenkonstruktiorl zusammen mit dem Boden oder einer am Boden befestigten Stützschiene. Die Tragstruktur weist die Formen von einem oder im Falle von zusätzlichen Nebenabschnitten von mehreren "u" auf.

Falls gewünscht, kann jeweils zwischen zwei benachbarten Haupt- und/oder Nebenabschnitten ein Seitenteil eingeschoben werden, das zu Markierungszwecken dienen kann. Insbesondere ist vorgesehen, jeweils an den beiden gegenüberliegenden kurzen Seiten des Grundkörpers zwei Kopfseitenteile mit entsprechender farbiger Markierung einzuschieben und dann fest durch Verschraubung zu fixieren. Auf diese Weise kann das Lagerungssystem lieferseitig schnell mit der speziellen Farbkodierung für eine bestimmte Schraubensorte angepasst werden.

Für eine gute Handhabbarkeit des Lagerungssystems weisen die Kopfseitenteile vorzugsweise Griffmulden auf und für die bessere Sterilisierbarkeit auch Durchtrittsöffnungen für die Reinigungsflüssigkeit und den Dampf.

Am oberen Ende weisen die Kopfseitenteile und auch die benachbarten Hauptabschnitte vorzugsweise Nuten zur Aufnahme eines einzuschiebenden Deckels auf. Um die Übersichtlichkeit über die in dem Lagerungssystem enthaltenen Implantate und Instrumente und deren Farbkodierung zu gewährleisten, ist der Großteil des Deckels vorzugsweise transparent und für eine gute Reinigung und Sterilisierbarkeit ebenfalls mit Durchtrittsöffnungen für die Reinigungsflüssigkeit und den Dampf versehen.

Der wesentliche Aspekt der vorliegenden Erfindung ist der tischartige Einsatz, der zur Aufnahme von Schraubenverpackungen dient. Dieser Tisch weist vier Beine und eine rechteckige oder quadratische horizontale Platte auf. An zwei einander gegenüberliegenden Seiten dieser Platte erstrecken sich mehrere längliche Öffnungen, die dazu dienen, durch Einschieben von Schraubenverpackungen aufzunehmen.

Die in dem Tisch aufzunehmenden Schraubenverpackungen sind In der EP 1 842 505 A2 im Detail beschrieben. Die Schraubenverpackungen sollten dreiteilig ausgebildet sein und eine Halteeinrichtung mit wenigstens einer Aufnahme für Schrauben, Stifte oder Klammern aufweisen, einen Deckel und eine an der Halteeinrichtung anbringbare Schutzabdeckung, welche aus der Halteeinrichtung nach unten herausragende Abschnitte der durch die Halteeinrichtung aufgenommenen Schrauben, Stifte oder Klammern umgibt.

Dadurch, dass das erfindungsgemäße Lagerungssystem keine Seitenwände aufweist, ist die seitliche Zugänglichkeit zu den Schraubenverpackungen und auch ein Wiederbefüllen durch Einschieben einer neuen Verpackung in das Lagerungssystem selbst mit darin angeordnetem tischartigen Einsatz möglich. Hierzu muss nur ein die Öffnungsmulden nach außen abschließender Schwenkarm ausgeschwenkt werden, so dass die Schraubenverpackungen aus den betreffenden länglichen Öffnungen herausgeschoben werden können,

Prinzipiell ist es ebenfalls möglich, die Schraubenverpackungen von oben in entsprechende Öffnungen in einen Einsatz einzustecken. Allerdings wird bei solch einer Ausführungsform auf die Führung beim Einschieben und die freie Zugänglichkeit von der Seite verzichtet.

In dem erfindungsgemäßen Lagerungssystem können auch andere Einsätze, beispielsweise für Nägel, Aneurysmenclips oder Gefäßclips angeordnet werden.

Der Tisch mit Füßen hat den Vorteil, dass er als solcher mit darin befindlicher Schraubenverpackung an einem anderen Ort aufgestellt werden kann.

Selbstrerständlich sind die erfindungsgemäßen Lagerungssysteme so ausgebildet, dass sie übereinander stapelbar sind.

Die Erfindung wird nachfolgend anhand von Ausführungsbeispielen näher beschreiben.

Es zeigen:
- **Fig. 1:**: eine perspektivische Ansicht des Grundkörpers des Lage- rungssystems,
- **Fig. 2:**: eine Seitenansicht des Grundkörpers aus Fig. 1 mit aufge- schobenem Deckel,
- **Fig. 3:**: eine perspektivische Ansicht des Lagerungssystems mit Grundkörper, Deckel und Einsatz zur Lagerung einer Schrau- benverpackung,

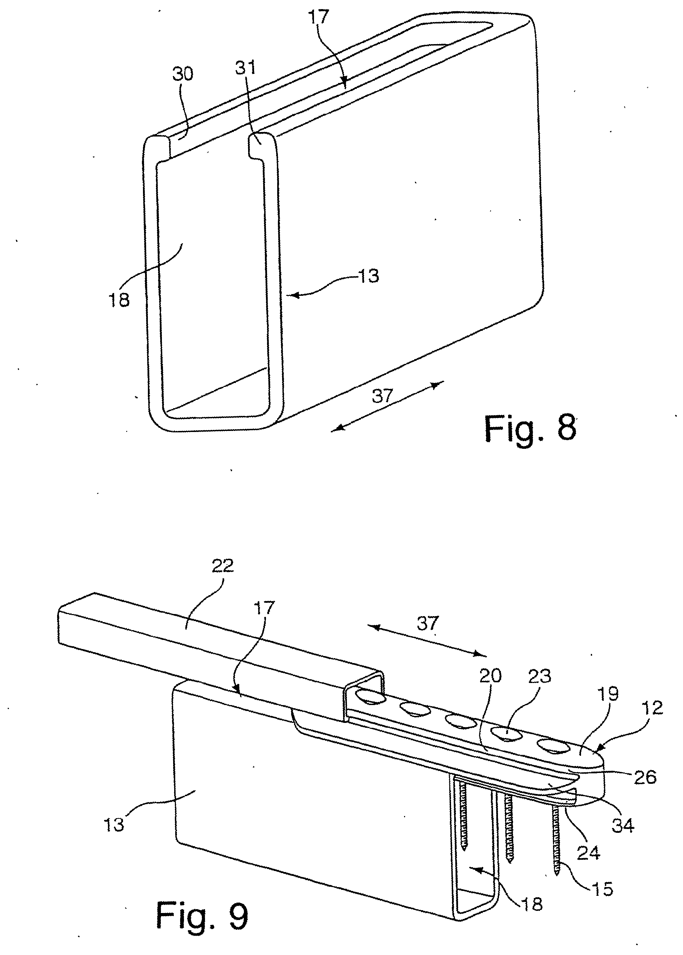

- **Fig. 4:**: einen Einsatz in den Grundkörper zur Lagerung der Schrau- benverpackung aus Fig. 5 bis 9 und
- **Fig. 5:**: eine Draufsicht auf den Einsatz zur Lagerung der Schrauben- verpackung (mit eingeschobenen Schraubenverpackungen),
- **Fig. 6:**: eine Seitenansicht der Schraubenverpackung im Schnitt mit Halteeinrichtung, Schraube, Schutzabdeckung und Deckel,
- **Fig. 7:**: eine Draufsicht auf die Halteeinrichtung mit zehn Bohrungen von oben,
- **Fig. 8:**: eine perspektivische Ansicht der Schutzabdeckung,
- **Fig. 9**:: eine perspektivische Seitenansicht der Schraubenverpackung mit Schrauben, teilweise eingeschobenem Deckel und teilweise eingeschobener Schutzabdeckung,

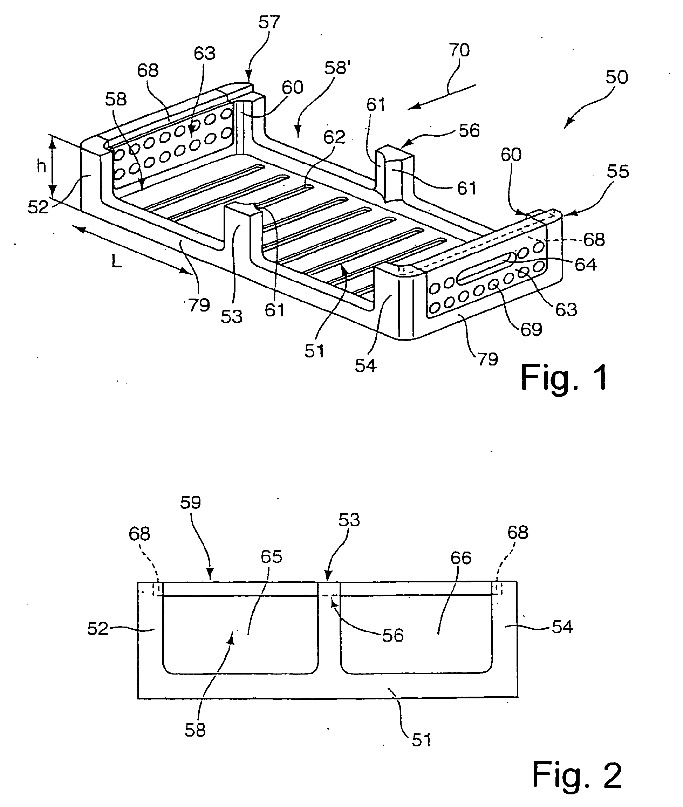

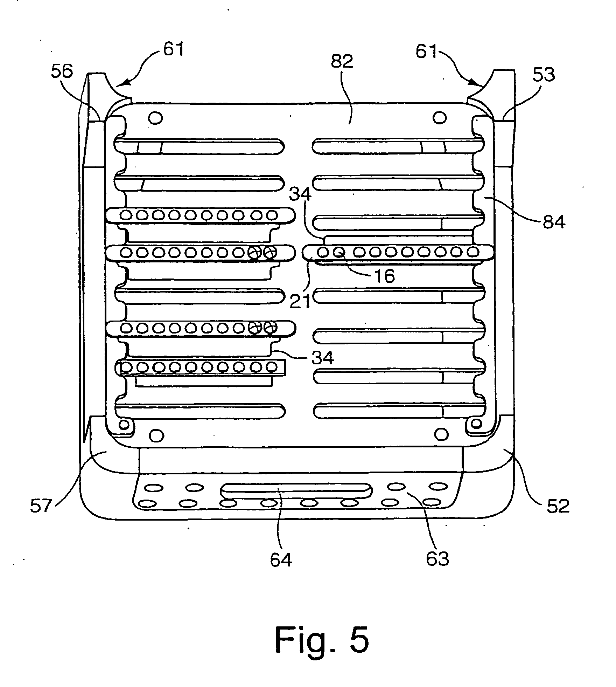

Das erfindungsgemäße Lagerungssystem ist in den Fig. 1 bis 5 näher erläutert.

Der Grundkörper 50 des Lagerungssystems weist eine Bodenplatte 51 auf, die eine Vielzahl von Öffnungen oder Rillen 62 zum Durchtritt der Reinigungs- und Sterilisierflüssigkeit/Dampf umfasst. Die Öffnungen und Rillen 62 sollten dabei nicht so groß sein, dass man von unten in das Lagerungssystem hineingreifen kann. Ansonsten sind auch größere Öffnungen oder Rillen 62 im Boden 51 in Bezug auf eine gute Reinig-/Sterilisierbarkeit durchaus erwünscht.

Die Bodenplatte 51 ist vorzugsweise aus Aluminium. Aluminium ist einerseits gegen die aggressiven, meist alkalischen Reinigungsflüssigkeiten hinreichend stabil, relativ leicht und ist zudem wärmeleitfähig, d.h. die Bodenplatte erwärmt sich während der Reinigung und Sterilisation hinreichend schnell und gibt auch die Wärme an die Halteabschnitte 53 ab.

Senkrecht zur Bodenplatte 51 sind vier, Hauptabschnitte 52, 54, 55, 57 angeordnet, die mit der Bodenplatte 51 starr verbunden sind. Diese Hauptabschnitte 52, 54, 56, 57 dienen als Führung für die auf der Bodenplatte zwischen den Hauptabschnitten 52, 54, 56, 57 anzuordnenden Einsätze, Tabletts oder Tische. Auch dienen die Hauptabschnitte 52, 54, 56, 57 zur Stabilisierung und - falls gewünscht - zur Aufnahme von einführbaren Seiten- oder Kopfseitenteilen 63. Vorzugsweise ist an jeder Ecke der Bodenplatte 51 jeweils ein Hauptabschnitt 52, 54, 55, 57 angeordnet und in Längsrichtung L befindet sich zwischen den beiden Hauptabschnitten 52, 54; 55, 57 jeweils noch ein weiterer Nebenabschnitt 53, 56, der zur Stabilisierung und Unterteilung des Grundkörpers 50 in zwei Bereiche 65, 66 dient. Hierfür weist jeder der beiden Nebenabschnitte 53, 56 jeweils zwei Führungsflächen 61 auf. Die Seitenflächen 58, 58' zwischen zwei Hauptabschnitten 52, 54 bzw. 55, 57 ist somit bis auf die Fläche des stegförmigen Hauptabschnittes 52, 54 bzw. 55, 57 und ggf. die Fläche des stegförmigen Nebenabschnittes 53 und der Fläche der Schiene 79 offen. Die Haupt- und Nebenabschnitte 52, 57 weisen verglichen mit der Gesamtlänge L eine geringe Breite auf.

Die Haupt- und Nebenabschnitte 52 bis 57 können auch untereinander über eine schmale Schiene 79 miteinander verbunden und die Schiene 79 an dem Boden 51 befestigt sein.

Insgesamt bilden die Haupt- und Nebenabschnitte, gegebenenfalls mit der Schiene 79 oder direkt mit dem Boden, eine Rahmen- oder Tragbalkenstruktur, insbesondere aus einem oder mehreren miteinander verbundenen U-förmigen Tragstrukturen (52, 79, 53; 53, 51, 54).

Die Haupt- und Nebenabschnitte 52, 53, 54, 55 und 57 weisen alle dieselbe Höhe h und an den Hauptabschnitten 52, 54, 55 und 57 und der senkrecht der Längsachse verlaufenden, nach innen weisenden Fläche des Kopfseitenteils 63 eine Nut 68 zum Einschieben des Deckels 59 auf.

Nachdem der Deckel 59 in Fig. 1 in Richtung des Pfeils 70 über den Nebenabschnitt 56 in die Nut 68 eingeschoben wird, ist die Höhe des Nebenabschnitts 56 gegenüber der anderen Abschnitte 52 - 55 und 57 verkürzt.

An der Stirnfläche des Lagerungssystems kann jeweils zwischen zwei Hauptabschnitten 52, 57 bzw. 54, 55 ein Kopfseitenteil 63 befestigt oder eingeschoben werden. Solche Kopfseitenteile 63 können herstellerseitig in verschiedenen Farben bereitgestellt werden, um dem Händler auf der Grundlage eines Grundkörpers 50 und verschiedener Kopfseitenteile 63 zu ermöglichen, für den Kunden das entsprechend einem bestimmten Schraubendurchmesser markierte Lagerungssystem auf einfache Weise bereit zu stellen. Der Händler kann das jeweils gewünschte Kopfseitenteil 63 in den Basiskörper 50 einschieben und dann durch Verschrauben, beispielsweise durch die Halteeinrichtungen, fixieren. Das Kopfseitenteil 63 weist ebenfalls Öffnungen 69 zur besseren Reinigung/Sterilisation und Griffmulden 64 auf.

Die Farbe des herstellerseitig einfügbaren Kopfseitenteils 63 korrespondiert mit der Farbe der Schraubenverpackung. Durch eine durchgängige farbige Markierung des Lagerungssystems und der Schraubenverpackungen kann sich der Arzt bei der Auswahl passender Schraubendurchmesser/Knochenplatten/Instrumente an der jeweiligen Farbe orientieren.

Die Kopfseitenteile 63 und die Abschnitte 52 bis 57 können aus Kunststoff oder Metall sein. Vorzugsweise sind sie aus PPSU.

Der Deckel 59 weist einen Rahmen 72 aus Metall auf und ist ansonsten im Wesentlichen transparent und aus Kunststoff. Öffnungen 71 im Deckel 59 ermöglichen einen Durchtritt der Reinigungs-/Sterilisierflüssigkeit. Durch die Transparenz des Deckels ist auch von oben die Einsicht in das Lagerungssystem möglich und beispielsweise durch die farbige Markierung überprüfbar, ob in dem Lagerungssystem die Schrauben passender Größe in der Schraubverpackung eingeschoben sind. Weiterhin weist der Deckel 59 eine Öffnung 73 auf, in die ein korrespondierender in dem Nebenabschnitt 53 angeordneter Stift 74 eingerastet werden kann.

Zur Aufnahme der nachfolgend beschriebenen Schraubenverpackungen ist ein zusätzlicher tischartiger Einsatz 80 vorgesehen, der von oben entlang der Führungsflächen 60, 61 der Haupt- und Nebenabschnitte 52, 53, 56, 57 oder 53, 54, 55, 56 auf den Boden 51 aufgestellt werden kann. Der tischartige Einsatz 80 weist vier Füße 81 und eine horizontale Platte 82 auf, in der mehrere längliche Öffnungen 83 vorgesehen sind, die zum seitlichen Einschieben der zuvor beschriebenen Schraubverpackungen 11 dienen. Zur Stabilisierung können die Füße miteinander an deren unterem Ende durch einen umfassenden Rahmen 86 verbunden sein. Im eingeschobenen Zustand liegen die Unterseite des Stegabschnitts 34 auf der horizontalen Platte 82 auf und die Randbereiche der horizontalen Platte 82 greifen in die Nut zwischen den Führungsmitteln 25, 24 und 28, 29 in der Halteeinrichtung 12 ein.

Auf den die Öffnungen 83 umfassenden Seiten ist an jeder Seite zusätzlich ein Schwenkarm 84 vorgesehen, um die in die länglichen Öffnungen 83 eingeschobenen Schraubverpackungen zu fixieren und ein Hängenbleiben oder die Gefahr von Verletzungen an den nach außen weisenden Öffnungen 83 zu verhindern.

Aufgrund der Zugänglichkeit des Schwenkarms 84 infolge der im Vergleich zum Stand der Technik fehlenden Seitenwand ist ein Öffnen und Schließen des Schwenkarms 84 und damit ein Einschieben und Entfernen der Schraubenverpackungen 11 und auch deren Schutzabdeckung 13 oder Deckel 22 in dem in dem Lagerungssystem befindlichen tischartigen Einsatz 80 möglich.

Nachdem die Öffnungen 83 von beiden Seiten des tischartigen Einsatzes 80 zugänglich sind, kann das Lagerungssystem von beiden Längsseiten be- und entladen werden.

Sind die Schutzabdeckung 13 und der Deckel 22 bei einer in dem tischartigen Einsatz 80 und in dem Lagerungssystem befindlichen Schraubenverpackungen 11 entfernt, so ist ein ungehinderter Durchtritt der Reinigungsflüssigkeit von der Seite an die frei in der Halteeinrichtung 13 hängenden Schraubenschäfte möglich.

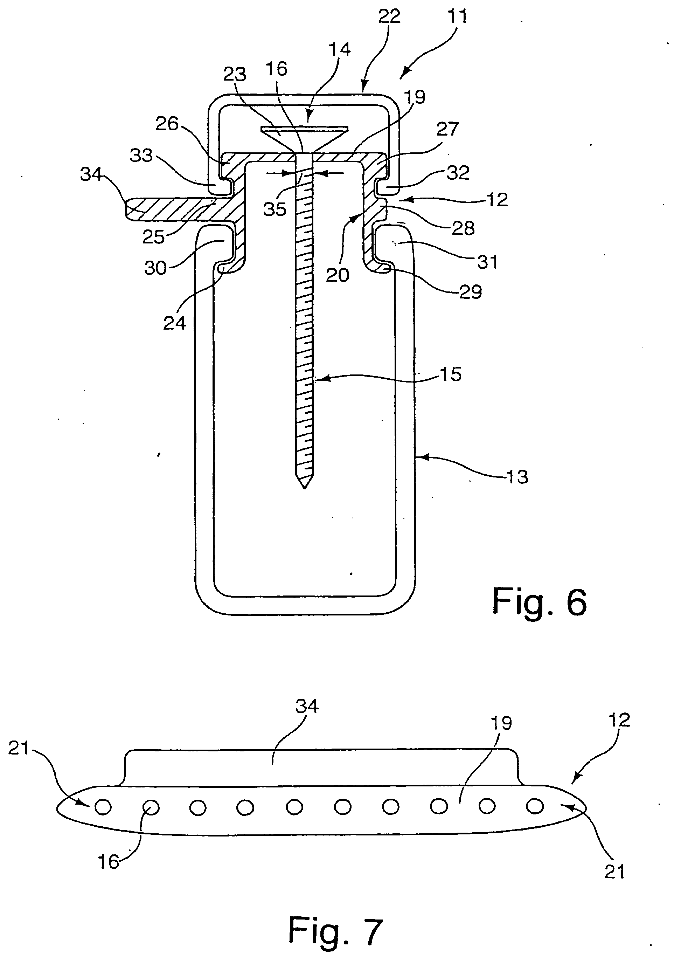

Die Schraubenverpackung 11 in **Fig. 6** umfasst eine Halteeinrichtung 12, eine Schutzabdeckung 13 und einen Deckel 22.

Die Halteeinrichtung 12 weist einen horizontalen Wandabschnitt 19 mit mehreren Bohrungen 16 auf, die dazu dienen, die in die Bohrungen 16 einsteckbaren Schrauben 14 zu halten. Hierzu ist der Durchmesser der Bohrungen 16 geringfügig größer als der Durchmesser 35 des Schraubenschaftes am Übergang zum Schraubenkopf 23, so dass die Schraube 14 mit der Unterseite des Schraubenkopfes 23 auf der nach oben weisenden Fläche des horizontalen Wandabschnitts 19 aufsitzt. Nach unten ragen Abschnitte 15 der Schrauben 14, insbesondere ein Großteil des Schraubenschaftes, aus der Halteeinrichtung 12 heraus.

Entlang der gesamten nach außen weisenden Kante des horizontalen Wandabschnitts 19 verläuft ein starr mit dem Wandabschnitt 19 verbundener vertikaler Wandabschnitt 20. Die Halteeinrichtung 12 ist somit annähernd quaderförmig und weist eine nach unten offene Fläche auf.

An den vertikalen Wandabschnitten 20 sind Führungsmittel 24, 25, 28, 29 vorgesehen, die dazu dienen, mit komplementären Führungsmitteln 30, 31 an der Schutzabdeckung 13 entsprechend einer Nut-Feder-Verbindung zusammenzuwirken. Auch sind an den vertikalen Wandabschnitten 20 Führungsmittel 25, 26, 27, 28 vorgesehen, die dazu dienen, mit komplementären Führungsmittel 32, 33 an dem Deckel 22 zu korrespondieren.

Die Dicke des vertikalen Wandabschnitts 20 und des horizontalen Wandabschnitts 19 beträgt vorzugsweise etwa 1 mm.

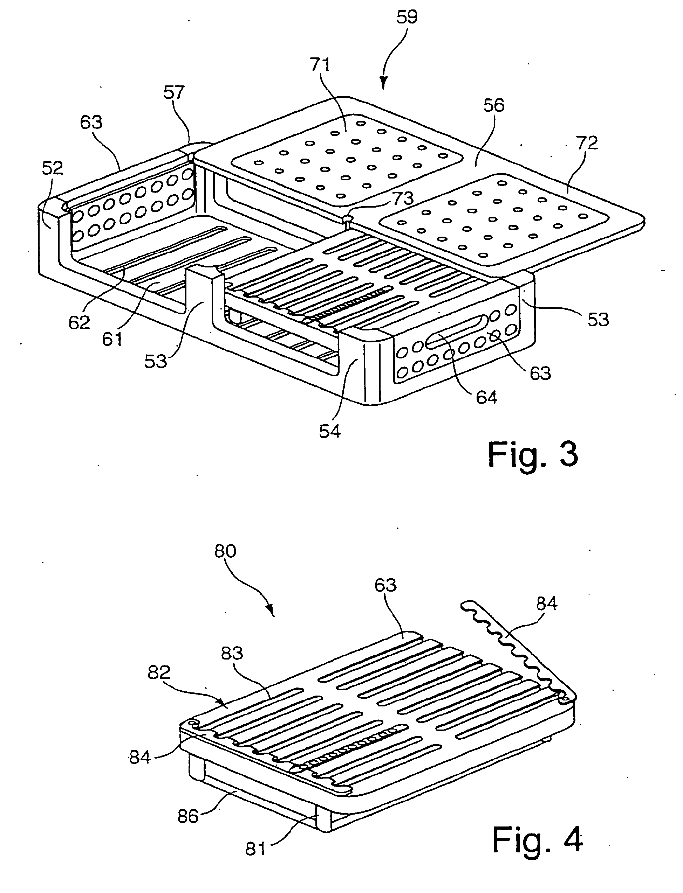

Die Schutzabdeckung 13 und der Deckel 22 werden vorzugsweise in Montage-/Verschieberichtung 37 in Fig. 4 auf die Halteeinrichtung 12 seitlich aufgeschoben.

Die Schutzabdeckung 13 und der Deckel 22, die schematisch in Figur 3 dargestellt sind, sind ein Gehäuse mit einer Öffnung 17, die dazu dient, mit der Halteeinrichtung 12 verbunden zu werden. Zusätzlich ist ein rechtwinklig zur Schiebe-Montageeinrichtung 37 verlaufender Wandabschnitt 18 freigeschnitten, der bei den erfindungsgemäßen Schiebemechanismen für Deckel und Schutzabdeckung das Einschieben der herausstehenden Schraubenköpfe und -schäfte ermöglicht.

Das eine Führungsmittel 25 geht in einen Stegabschnitt 34 über, der dazu dient, an seiner Oberseite mit den jeweiligen Chargen-/LOT-Nummern beschriftet zu werden.

Dadurch, dass keine direkte Verbindung zwischen dem vertikalen Wandabschnitt 20 und den herabhängenden Schraubenschäften 15 besteht, wird verhindert, dass während der Operation vom Schraubenzieher herabtropfendes Blut an die Schraubenschafte gelangen kann. Zudem wird ein Herunterlaufen von Blut auf einer Seite der Vorrichtung auch noch durch den Stegabschnitt 34 verhindert.

Soll eine Schraubenverpackung in der horizontalen Platte 82 des Lagerungssystems gelagert werden, so wird die Schraubenverpackung mit Schrauben 14, ggf. mit Deckel 22, aber ohne Schutzabdeckung 13 seitlich in eine längliche Öffnung 83 in der horizontalen Platte 82 eingeschoben, so dass die Randbereiche der Platte 82 entlang der länglichen Öffnung 83 entsprechend der Führungsmittel 30, 31 in der Schutzabdeckung 13 in Figur 1 in der Nut zwischen den Führungsmitteln 25, 24 und 28, 29 aufgenommen werden.

Die nach unten weisende Seite des Stegabschnitts 34 kann weiterhin dann, wenn die Vorrichtung in einem entsprechenden Lagerungssystem gelagert ist, als Auflagefläche dienen.

Ebenfalls zur Auflage in einem entsprechenden Lagerungssystem können die nach unten weisenden Flächen der beiden Außenflächen 21 in dem horizontalen Wandabschnitt 19 dienen.

Die Größe der Vorrichtung ist von der Anzahl der darin aufzubewahrenden Schrauben und deren Durchmesser abhängig.

Bei einer Anzahl von 10 Schrauben und einem Durchmesser der Bohrungen 16 für die Schrauben von ca. 2,5 mm beträgt die Gesamtlänge der Vorrichtung ca. 55 mm, die Höhe ca. 30 mm und die Tiefe ca. 8 mm.

Bei dieser Ausführungsform entspricht der Abstand der Bohrungen 16 zueinander in etwa dem Durchmesser der Bohrung.

## Patentansprüche

1. Lagerungssystem für Implantate, weiches Lagerungssystem eine Bodenplatte (51) und vier senkrecht zur Bodenplatte (51) verlaufende stegförmige Hauptabschnitte (52, 54; 55, 57) und einen Deckel (59) aufweist, wobei zwei benachbarte Hauptabschnitte (52, 54; 55, 57) eine Seitenfläche (58, 58') bilden, die im Wesentlichen offen oder freigeschnitten ist und der Prozentsatz der offenen oder freigeschnittenen Seitenfläche zur gesamten Seitenfläche des Lagerungssystems wenigstens 30 % beträgt,
**dadurch gekennzeichnet,**
**dass** die Hauptabschnitte (52, 54; 55, 57) und ggf. seitlich vorhandenen Nebenabschnitte (53, 56) zusammen mit dem Boden (51) oder einer an dem Boden (51) befestigten Stütze (79) einen Rahmen oder eine Tragbalkenkonstrukäon bilden und
**dass** das Lagerungssystem einen tischartigen Einsatz (80) zur Aufnahme von Verpackungen von Schrauben, Nägeln, Aneurysmenclips oder Gefäßclips umfasst und der tischartige Einsatz (80) eine horizontale Platte (82) mit mehreren länglichen Öffnungen (83) und wenigstens vier Füße (81) aufweist,
wobei durch die Öffnung oder den Freischnitt zwischen den beiden benachbarten Hauptabschnitten (52, 54; 55, 57) eine seitliche Zugänglichkeit und ein guter Durchtritt der Reinigungsflüssigkeit in der Spülmaschine und des Dampfes selbst dann gewährleistet Ist, wenn der tischartige Einsatz in dem Lagerungssystem angeordnet ist.

2. Lagerungssystem nach Anspruch 1, **dadurch gekennzeichnet, dass** wenigstens zwei jeweils zwischen zwei benachbarten Hauptabschnitten (52, 54; 55, 57) aufgespannte Seitenflächen (58, 58') im Wesentlichen offen oder freigeschnitten sind.

3. Lagerungssystem nach Anspruch 2, **dadurch gekennzeichnet, dass** die wenigstens zwei im Wesentlichen offenen oder freigeschnittenen Seitenflächen (58; 58') auf den einander gegenüberliegenden Seiten angeordnet sind.

4. Lagerungssystem nach einem der Ansprüche 1 bis 3, **dadurch gekennzeichnet, dass** die Seitenfläche(n) zusätzliche stegförmige Nebenabschnitte (53, 56) aufweist.

5. Lagerungssystem nach Anspruch 4, **dadurch gekennzeichnet, dass** die Nebenabschnitte (53, 56) eine geringe Breite aufweisen und als Stützen für Deckel und zur Lagesicherung der Einlagen dienen.

6. Lagerungssystem nach einem der Ansprüche 1 bis 5, **dadurch gekennzeichnet, dass** die nach innen weisenden Flächen (60, 61) der Haupt- und Nebenabschnitte (52, 53, 54, 55, 56, 57) als Führungsflächen für Einlegeböden, Tabletts oder Einlegetische ausgebildet sind.

7. Lagerungssystems nach einem der Ansprüche 1 bis 6, **dadurch gekennzeichnet, dass** die Hauptabschnitte (52, 54, 55, 57) Mittel (68) zur Aufnahme oder zum Einschieben des Deckels (59) aufweisen.

8. Lagerungssystem nach Anspruch 7, **dadurch gekennzeichnet, dass** die Hauptabschnitte (52, 54, 55, 57) Mittel zur Aufnahme eines Kopfseitenteils (63) aufweisen, das vorzugsweise in die Hauptabschnitte (52, 57; 54, 55) einschiebbar ist.

9. Lagerungssystem nach Anspruch 8, **dadurch gekennzeichnet, dass** das Kopfseitenteil (63) mit den Hauptabschnitten (52, 57; 54, 55) fest verbindbar ist, insbesondere verschraubbar ist, das Kopfseitenteil (63) Griffmulden (64) aufweist und vorzugsweise farbig ist.

10. Lagerungssystem nach Anspruch 1, **dadurch gekennzeichnet, dass** die länglichen Öffnungen (83) von den beiden Längsseiten des Lagerungssystems zugänglich sind.

11. Lagerungssystem nach Anspruch 1 oder 10, **dadurch gekennzeichnet, dass** der Einsatz (80) zusätzlich wenigstens einen Schwenkarm (84) aufweist, der von wenigstens einer Längsseite des Lagerungssystems bedienbar ist.

12. Lagerungssystem nach einem der vorangegangenen Ansprüche, **dadurch gekennzeichnet, dass** der Prozentsatz der offenen oder freigeschnittenen Seitenfläche zur gesamten Seitenfläche des Lagerungssystems wenigstens 40 % und insbesondere mehr als 50 % beträgt.

13. Lagerungssystem nach einem der Ansprüche 1 bis 12, **dadurch gekennzeichnet, dass** wenigstens Teile des Lagersystems farbkodiert sind.

## Claims

1. Storage system for implants, which storage system has a base plate (51), four web-shaped main sections (52, 54; 55, 57) extending perpendicular to the base plate (51), and a lid (59), where two adjacent main sections (52, 54; 55, 57) form a side surface (58, 58') which is substantially open or cut free, and the percentage of the open or cut-free side surface relative to the total side surface of the storage system is at least 30%, **characterized in that** the main sections (52, 54; 55, 57) and, if appropriate, subsidiary sections (53, 56) present at the sides form, together with the base (51), or with a support (79) secured on the base (51), a frame or a supporting beam structure, and **in that** the storage system comprises a table-like insert (80) for receiving packages of screws, nails, aneurysm clips or vascular clips, and the table-like insert (80) has a horizontal plate (82) with several elongate openings (83) and at least four feet (81), and the opening or cut-out between the two adjacent main sections (52, 54; 55, 57) ensures lateral accessibility and an efficient passage of the cleaning liquid in the washer and of the steam even when the table-like insert is arranged in the storage system.

2. Storage system according to Claim 1, **characterized in that** at least two side surfaces (58, 58') included in each case between two adjacent main sections (52, 54; 55, 57) are substantially open or cut free.

3. Storage system according to Claim 2, **characterized in that** the at least two substantially open or cut-free side surfaces (58, 58') are arranged on the sides facing each other.

4. Storage system according to one of Claims 1 to 3, **characterized in that** the side surface(s) has/have additional web-shaped subsidiary sections (53, 56).

5. Storage system according to Claim 4, **characterized in that** the subsidiary sections (53, 53') have a small width and serve as supports for lids and for securing the position of the inserts.

6. Storage system according to one of Claims 1 to 5, **characterized in that** the inwardly facing surfaces (60, 61) of the main and subsidiary sections (52, 53, 54, 55, 56, 57) are designed as guide surfaces for insert bases, trays or insert tables.

7. Storage system according to one of Claims 1 to 6, **characterized in that** the main sections (52, 54, 55, 57) have means (68) for receiving or pushing in the lid (59).

8. Storage system according to Claim 7, **characterized in that** the main sections (52, 54, 55, 57) have means for receiving a head side part (63), which can preferably be pushed into the main sections (52, 57; 54, 55).

9. Storage system according to Claim 8, **characterized in that** the head side part (63) can be securely connected, in particular screwed, to the main sections (52, 57; 54, 55), the head side part (63) having grip recesses (64) and preferably being coloured.

10. Storage system according to Claim 1, **characterized in that** the elongate openings (83) are accessible from the two lengthwise sides of the storage system.

11. Storage system according to Claim 1 or 10, **characterized in that** the insert (80) additionally has at least one pivot arm (84), which can be operated from at least one lengthwise side of the storage system.

12. Storage system according to one of the preceding claims, **characterized in that** the percentage of the open or cut-free side surface relative to the total side surface of the storage system is at least 40% and in particular more than 50%.

13. Storage system according to one of Claims 1 to 12, **characterized in that** at least parts of the storage system are colour-coded.

## Revendications

1. Système de stockage pour implants, lequel système de stockage présente une plaque de fond (51) et quatre portions principales en forme de nervures (52, 54 ; 55, 57) s'étendant perpendiculairement à la plaque de fond (51) et un couvercle (59), deux portions principales adjacentes (52, 54 ; 55, 57) formant une surface latérale (58, 58'), qui est essentiellement ouverte ou découpée, et le pourcentage de surface latérale ouverte ou découpée par rapport à la surface latérale totale du système de stockage valant au moins 30%, **caractérisé en ce que** les portions principales (52, 54 ; 55, 57) et éventuellement des portions auxiliaires prévues latéralement (53, 56) formant, conjointement avec le fond (51) ou avec un support (79) fixé sur le fond (51), un cadre ou une construction de poutre portante et **en ce que** le système de stockage comprend un insert de type table (80) pour recevoir des emballages de vis, clous, pinces pour anévrismes ou pinces pour récipients et l'insert de type table (80) présente une plaque horizontale (82) avec plusieurs ouvertures oblongues (83) et au moins quatre pieds (81), l'ouverture ou la découpure entre les deux portions principales adjacentes (52, 54 ; 55, 57) permettant une accessibilité latérale et un bon passage du liquide de nettoyage dans la machine de rinçage et de la vapeur proprement dite, lorsque l'insert de type table est disposé dans le système de stockage.

2. Système de stockage selon la revendication 1, **caractérisé en ce qu'**au moins deux surfaces latérales (58, 58') tendues entre deux portions principales adjacentes (52, 54 ; 55, 57) sont essentiellement ouvertes ou découpées.

3. Système de stockage selon la revendication 2, **caractérisé en ce que** les au moins deux surfaces latérales (58, 58') essentiellement ouvertes ou découpées sont disposées sur les côtés mutuellement opposés.

4. Système de stockage selon l'une quelconque des revendications 1 à 3, **caractérisé en ce que** la ou les surfaces latérales présentent des portions auxiliaires supplémentaires en forme de nervures (53, 56).

5. Système de stockage selon la revendication 4, **caractérisé en ce que** les portions auxiliaires (53, 56) présentent une plus faible largeur et servent de support pour des couvercles et pour la fixation en position des inserts.

6. Système de stockage selon l'une quelconque des revendications 1 à 5, **caractérisé en ce que** les surfaces tournées vers l'intérieur (60, 61) des portions principales et auxiliaires (52, 53, 54, 55, 56, 57) sont réalisées sous forme de surfaces de guidage pour des fonds d'insertion, des tablettes ou des tables d'insertion.

7. Système de stockage selon l'une quelconque des revendications 1 à 6, **caractérisé en ce que** les portions principales (52, 54, 55, 57) présentent des moyens (68) pour recevoir ou pour enfoncer le couvercle (59).

8. Système de stockage selon la revendication 7, **caractérisé en ce que** les portions principales (52, 54, 55, 57) présentent des moyens pour recevoir une partie latérale de tête (63), qui peut être enfoncée de préférence dans les portions principales (52, 57 ; 54, 55).

9. Système de stockage selon la revendication 8, **caractérisé en ce que** la partie latérale de tête (63) peut être connectée fixement aux portions principales (52, 57 ; 54, 55) notamment par vissage, **en ce que** la partie latérale de tête (63) présente des creux de préhension (64) et de préférence est colorée.

10. Système de stockage selon la revendication 1, **caractérisé en ce que** les ouvertures oblongues (83) sont accessibles depuis les deux côtés longitudinaux du système de stockage.

11. Système de stockage selon la revendication 1 ou 10, **caractérisé en ce que** l'insert (80) présente en outre au moins un bras pivotant (84) qui peut être commandé depuis au moins un côté longitudinal du système de stockage.

12. Système de stockage selon l'une quelconque des revendications précédentes, **caractérisé en ce que** le pourcentage de la surface latérale ouverte ou découpée par rapport à la surface latérale totale du système de stockage vaut au moins 40% et notamment plus de 50%.

13. Système de stockage selon l'une quelconque des revendications 1 à 12, **caractérisé en ce qu'**au moins des parties du système de stockage sont munies d'un codage de couleur.
